# EUROPEAN PATENT SPECIFICATION

(11) **EP 4 713 704 B1**
(45) Date of publication and mention of the grant of the patent: **09.12.2026**
(21) Application number: 25710121.2
(22) Date of filing: 12.02.2025
(51) Int. Cl.: G01R 33/485, A61B 5/055, G01R 33/56

(54) **MAGNETIC RESONANCE METHOD, COMPUTER PROGRAM PRODUCT AND MAGNETIC RESONANCE IMAGING MACHINE**
MAGNETRESONANZVERFAHREN, COMPUTERPROGRAMMPRODUKT UND MAGNETRESONANZBILDGEBUNGSMASCHINE
PROCÉDÉ DE RÉSONANCE MAGNÉTIQUE, PRODUIT PROGRAMME D'ORDINATEUR ET MACHINE D'IMAGERIE PAR RÉSONANCE MAGNÉTIQUE

(30) Priority: 13.02.2024 IT 202400003025
(43) Date of publication of application: 25.03.2026
(73) Proprietor: Università degli Studi di Torino, 10124 Torino (IT); Politecnico Di Torino, 10129 Torino (IT)
(72) Inventor: DELLI CASTELLI, Daniela, 10124 TORINO (IT); SCELLATO, Giuseppe, 10129 TORINO (IT); AIME, Silvio, 10124 TORINO (IT); VASSALLO, Giulia, 10124 TORINO (IT); GARELLO, Francesca, 10124 TORINO (IT); FIORUCCI, Cecilia, 10124 TORINO (IT)
(74) Representative: Studio Torta S.p.A.
(86) International application number: PCT/IB2025/051441
(87) International publication number: WO 2025/172844

(56) References cited:
- MORA B ET AL: "31P SATURATION TRANSFER AND PHOSPHOCREATINE IMAGING IN THE MONKEY BRAIN", PROCEEDINGS OF THE NATIONAL ACADEMY OF SCIENCES, NATIONAL ACADEMY OF SCIENCES, vol. 88, no. 19, 1 October 1991 (1991-10-01), pages 8372 - 8376, XP000979303, ISSN: 0027-8424, DOI: 10.1073/PNAS.88.19.8372
- VALKOVIC LADISLAV ET AL: "In-vivo31P-MRS of skeletal muscle and liver: A way for non-invasive assessment of their metabolism", ANALYTICAL BIOCHEMISTRY, ACADEMIC PRESS, AMSTERDAM, NL, vol. 529, 21 January 2017 (2017-01-21), pages 193 - 215, XP085063686, ISSN: 0003-2697, DOI: 10.1016/J.AB.2017.01.018
- VASSALLO GIULIA ET AL: "31P ParaCEST: 31P MRI-CEST Imaging Based on the Formation of a Ternary Adduct between Inorganic Phosphate and Eu-DO3A", vol. 61, no. 49, 29 November 2022 (2022-11-29), Easton , US, pages 19663 - 19667, XP093194245, ISSN: 0020-1669, Retrieved from the Internet <URL:https://pubs.acs.org/doi/pdf/10.1021/acs.inorgchem.2c03329> DOI: 10.1021/acs.inorgchem.2c03329

## Description

### Technical Field

The present invention relates to a magnetic resonance method for analyzing metabolic processes, in particular enzymatic processes.

Advantageously, the present invention also relates to a program product for controlling the execution of a magnetic resonance method for analyzing metabolic processes, in particular enzymatic processes.

Advantageously, the present invention also relates to a magnetic resonance imaging machine for analyzing metabolic processes, in particular enzymatic processes.

### Background of the Invention

Different types of metabolic processes take place in living beings. An example of this is glycolysis, which consists of a series of chemical reactions that take place in the cytoplasm of cells and transform glucose (a molecule with 6 carbon atoms) into two pyruvate molecules (a molecule with 3 carbon atoms), producing energy in the form of two molecules of adenosine triphosphate (ATP) and two molecules of nicotinamide adenine dinucleotide (NADH). The pyruvate produced can be further utilized in different metabolic pathways depending on oxygen availability. In particular, in the presence of oxygen, pyruvate enters the Krebs cycle and its transformation by a cascade of reactions leads to oxidative phosphorylation, whereas in the absence of oxygen, pyruvate is converted to lactate. It is known that glycolysis is strongly deregulated in cancer cells. In fact, the Warburg effect describes a phenomenon observed in many cancer cells, which perform anaerobic glycolysis as an energy source instead of oxidative phosphorylation, even in the presence of oxygen. This metabolic shift has a negative impact on ATP production: anaerobic glycolysis produces only two ATP molecules per molecule of metabolized glucose, whereas oxidative phosphorylation produces 38. However, this choice allows cancer cells to generate precursors for the biosynthesis of macromolecules, which are needed to support the rapid proliferation process.

In addition to oncology, glycolysis has been shown to be abnormal in peripheral cells in major neurodegenerative diseases: Alzheimer's, Parkinson's and in amyotrophic lateral sclerosis. These metabolic alterations occur at an early stage of the disease, so their observation allows the disease to be studied at an early stage.

Another metabolic pathway often deregulated in various diseases is gluconeogenesis, which involves a series of enzymatic reactions that convert precursor molecules, such as pyruvate, into glucose. In humans and animals, this process occurs mainly in the liver and to a lesser extent in the kidneys. The enzymes involved in gluconeogenesis are regulated so that they are active when the body needs to increase blood glucose levels.

Gluconeogenesis is a complementary process to glycolysis, which is responsible for the breakdown of glucose to produce energy. The ability to convert non-glucose molecules into glucose is crucial for ensuring a constant supply of glucose to the brain and other tissues that depend on this sugar as an energy source. Many reactions of gluconeogenesis involve the same phosphorylated substrates as glycolysis. In the liver, glycolysis and gluconeogenesis are regulated in such a way that when one pathway is active, the other is quiescent, and vice versa. Examples of enzymes that characterise glycolysis as opposed to gluconeogenesis are phosphofructokinase (which catalyzes the transformation of the fructose 6-phosphate molecule into the fructose 1,6-bisphosphate molecule in the early stages of glycolysis) and fructose 1,6-bisphosphatase (which catalyzes the transformation of the fructose 1,6-phosphate molecule into the fructose 6-phosphate molecule in the latter stages of gluconeogenesis). Deregulation of gluconeogenesis can occur in various pathological conditions or diseases, often associated with genetic defects, hormonal dysfunction or metabolic disorders. Among the main diseases with which deregulation of gluconeogenesis is associated is type 2 diabetes mellitus.

For example, increased gluconeogenesis in the liver of patients with type 2 diabetes is known to be a major contributor to hyperglycaemia and subsequent diabetic organ damage. Insulin is a key hormone that inhibits gluconeogenesis and insulin resistance is a hallmark of type 2 diabetes. Observing the deregulation of gluconeogenesis is therefore of great importance for developing and monitoring the effectiveness of therapies for type 2 diabetes. Moreover, in the liver, glucose metabolism (e.g. glycolysis, gluconeogenesis and the pentose phosphate pathway) is closely related to lipid metabolism. These catabolic and anabolic pathways are difficult to separate and their deregulation is associated with the pathogenesis of the most common liver diseases such as hepatic steatosis (fatty liver). Non-alcoholic fatty liver disease (NAFLD) is the most common liver disease in the world, affecting more than 25% of the world's population. The spectrum of NAFLD ranges from mild steatosis to steatohepatitis with fibrosis with potential development of cirrhosis, which is now the main indication for liver transplantation in women and individuals over 65 years of age. Gluconeogenesis has been shown to be one of the metabolic pathways deregulated by this disease. The development of increasingly detailed noninvasive assessment tools would enable advances in the diagnosis of NAFLD leading to better stratification of patients on the basis of risk of disease evolution.

In recent years, various imaging techniques have been successfully used to view and characterize diseases associated with deregulated metabolism. The combination of these protocols and imaging techniques aimed at viewing *in vivo* abnormalities at the level of the main metabolic pathways is called Metabolic Imaging.

Mora et al. (PNAS, Vol. 88, 8372-8376, 1991) reports a study using ³¹P magnetic resonance imaging with selective excitation to map phosphocreatine (PCr) distribution and evaluate creatine kinase (CK) activity in the brains of macaque monkeys, both at rest and during visual stimulation. By selectively saturating the γ-phosphate of ATP and measuring resulting changes in PCr signal intensity, the researchers quantified magnetization transfer and estimated CK flux, producing spatial PCr and CK activity maps that revealed heterogeneous metabolite distribution across brain regions. Visual stimulation experiments showed localized decreases in PCr concentrations in some trials-typically in or near visual cortical areas-suggesting increased local ADP levels and heightened metabolic demand linked to oxidative phosphorylation and glycolysis, although the response varied between animals. The study demonstrates the feasibility of noninvasive in vivo metabolic imaging to assess regional brain energy metabolism and its modulation by sensory activation.

With regard to the observation of deregulation of glycolysis, a number of MRI methodologies are being developed that require the use of contrast agents such as, for example, hyperpolarized contrast agents, or deuterated molecules or CEST agents.

A technique applied in the clinical setting is FDG-PET which is a Positron Emission Tomography technique using fluorodeoxyglucose (FDG) labelled with the radioactive isotope ¹⁸F as a tracer. FDG is an analog of the glucose molecule. Once administered, FDG uses the same transporters as glucose to enter cells where it is phosphorylated. Cells with a higher-than-normal glucose consumption will be the ones from which the largest amount of signal that is detected during measurements will be derived. The resulting PET images illustrate the distribution and concentration of FDG within the body, providing information on cellular metabolic activity.

While the sensitivity is very high, there are some disadvantages to using the FDG-PET technique. Firstly, it has a very low spatial resolution, often necessitating the use of hybrid techniques in combination with magnetic resonance imaging (MRI) or computed tomography (CT), which allow the PET signal to be superimposed on images that render greater morphological detail. Moreover, PET cannot differentiate between different molecules, as positron emission from different radionuclides always produces the same response. Another disadvantage concerns the production of radionuclides, which must be carried out in close proximity to the hospital where the test will be performed. In addition, false positives may occur, necessitating careful interpretation of the images in combination with other clinical information.

The FDG-PET technique also has a limitation related to the inability to observe the metabolic process dynamically. A further disadvantage lies in exposing the patient to radiation.

There is therefore a need in the industry for further improvement of methods and machines for analyzing metabolic processes.

In particular, there is a need for methods that do not require the use of exogenous molecules, thus eliminating the possible health risks associated with the use of contrast agents.

### Description of the Invention

The aim of the present invention is to provide an improved method that enables the analysis of metabolic processes.

A further aim of the present invention is to provide a computer program product to control the execution of a method for analyzing metabolic processes.

Another aim of the present invention is to provide a magnetic resonance imaging machine for analyzing metabolic processes.

The above aims are achieved by the present invention, since it relates to the method as defined in independent claim 1. Preferred embodiments are protected in the respective dependent claims.

The above aims are achieved by the present invention, since it relates to a computer program product as defined in claim 13.

The aforesaid aims are achieved by the present invention, since it relates to a magnetic resonance imaging machine according to claim 14.

### Brief Description of the Drawings

The present invention will now be described with reference to the accompanying drawings related to some nonlimiting embodiments thereof, in which:
- Figure 1 schematically shows an MRI machine, with parts removed for clarity;
- Figure 2 shows the glycolytic process;
- Figures 3a and 3b show magnetic resonance spectra;
- Figures 4 to 6 show z spectra of magnetic resonance measurements with the CEST mode; and
- Figures 7A to 7D show an anatomical MRI image together with additional information obtainable by the method of the present invention.

### Preferred Embodiments of the Invention

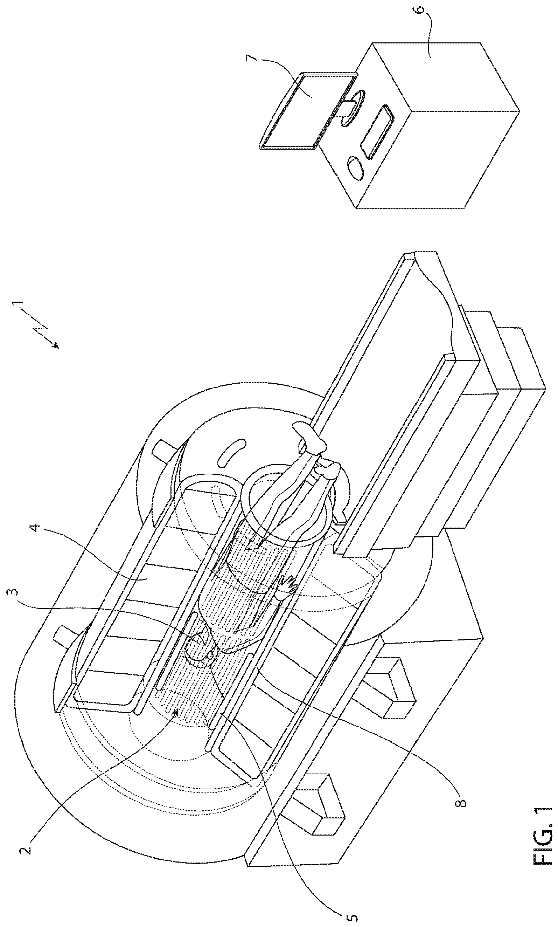

With reference to Figure 1, number 1 denotes a magnetic resonance machine, more specifically a magnetic resonance imaging machine, comprising:
- a measurement space 2 to receive a biological object 3;
- a generating device 4, e.g. having a permanent magnet and/or an electromagnet and/or a superconducting magnet, configured to generate a static magnetic field;
- an emitter device 5 configured to irradiate the biological object 3 with a radiofrequency field (rf); and
- an acquisition device configured to acquire nuclear magnetic resonance response signals; and
- a control device 6 configured to control the generating device 4 and/or the emitter device 5 and/or the acquisition device to perform magnetic resonance measurements.

Preferably but not necessarily, the MRI machine 1 may also comprise a display device 7, e.g. a screen, configured to display MRI information, such as spectra, MRI images etc.

In more detail, the generating device 4 can be configured to generate a static magnetic field of at least 3 T or at least 7 T.

In more detail, the emitter device 5 can be configured to irradiate biological object 3 with an RF field, for example, with an intensity (B₁) of the magnetic field that can vary from 0.5 µT to 12.0 µT, more specifically from 1 µT to 5 µT.

More specifically, the emitter device 5 can be configured to emit radiofrequency pulses.

The emitter device 5 may comprise one or more emission coils and a power unit to generate the radiofrequency field, more specifically radiofrequency field pulses.

In more detail, the acquisition device can be configured to process the response signals.

Preferably but not necessarily, the acquisition device comprises receiving coils to detect the response signals.

More specifically, the emitter coils also function as receiving coils. In other words, the MRI machine comprises coils that define both a portion of the emitter device 5 and a portion of the acquisition device. In other words, the coils act as a transmitter or receiver depending on the specific function they have to perform at a given point in time.

The magnetic resonance imaging machine 1 may also comprise an analysis device configured to analyze the response signals, e.g. to extract information from the response signals and/or to form magnetic resonance images.

The magnetic resonance imaging machine 1 may also comprise a magnetic gradient generating device 8 configured to emit a magnetic field, in particular to induce a local variation of the static magnetic field. In particular, the magnetic gradient generating device 8 is configured to obtain spatial information (which in turn allows for three-dimensional information).

The magnetic resonance imaging machine 1 is configured to perform measurements in CEST (Chemical Exchange Saturation Transfer) mode.

In particular, CEST mode is based on the saturation of magnetic resonance signals belonging to nuclei of atoms that are in exchange with other molecules that are excited to obtain the response signals on which the saturation transfer will be observed. Saturation transfer results in a change in response signals.

In traditional CEST mode, response signals from the water in a sample are recorded. The amount of saturation transfer depends directly on the mole fraction between the nuclei irradiated by the RF field and the nuclei of the acquired signal to which saturation is transferred. The minimum value of the mole fraction permitting the display of a measurable saturation transfer effect, when considering only one irradiated nucleus per molecule and under exchange conditions in a range compatible with the CEST experiment (i.e. the value of the exchange rate expressed as the chemical exchange constant in sec⁻¹ must be lower than the frequency separation between the magnetic resonance signals belonging to the two exchanging nuclei) falls in the range of 10⁻⁵-10⁻⁴. Therefore, when the nuclei acquired are the protons of water, whose concentration in a biological sample is 111.2M, it is not possible to detect concentrations of mobile protons belonging to the target molecule below about 1 mM per single proton.

The Applicant found that one way to be able to lower the detection threshold of the CEST technique and observe target molecules at concentrations below 1mM, whose response signals are below the signal-to-noise ratio (SNR - i.e. not detectable in a static magnetic field below 7T) is not to observe the response signals of water. In other words, it can no longer be water that defines the acquired signal, but a signal belonging to a molecule that is still endogenous but has a lower concentration of observable nuclei than water in the biological object.

The Applicant has found that the saturation of the signal belonging to nuclei of phosphorus atoms ³¹P bound in target molecules to obtain a saturation transfer to a signal belonging to nuclei of phosphorus atoms ³¹P of endogenous molecules which are in saturation transfer with saturated phosphorus ³¹P allows the study of metabolic reactions. According to the present invention enzymatic reactions belonging to glycolysis and/or gluconeogenesis are the object of study.. In general, the method proposed here applies to the direct analysis in the biological object 3 of enzymatic reactions involving kinases and phosphatases.

In addition, the method described here can also generate saturation transfer measurements that constitute a fingerprint region (i.e. a reference measurement) for each area of the biological object under investigation. The other measurements resulting from the application of the method can then be compared with the reference measurement, i.e. the fingerprint.

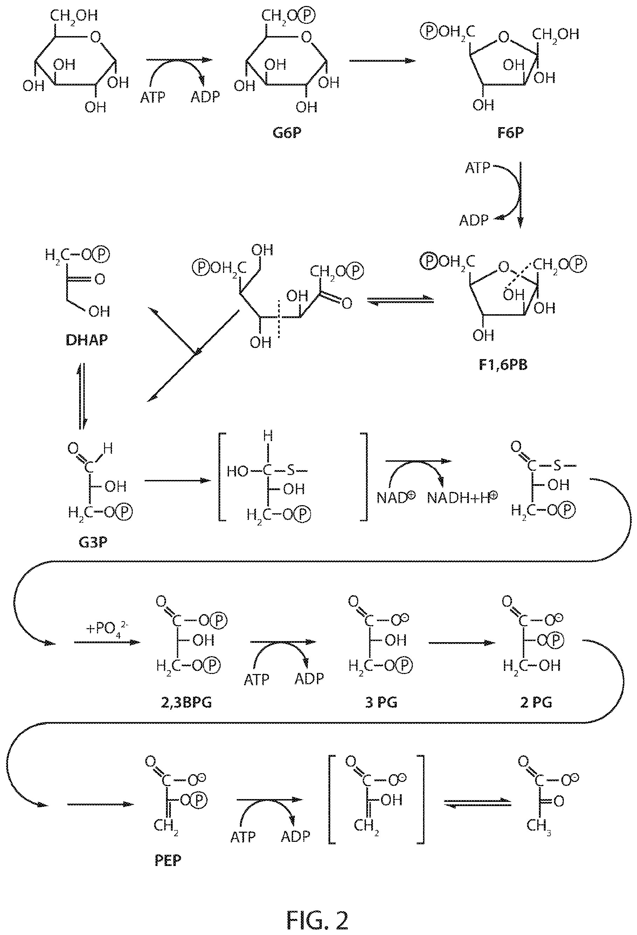

As can be seen from Figure 2, during the glycolysis process, a number of possible target molecules containing a phosphorus can be identified, such as glucose-6-phosphate (G6P), fructose-6-phosphate (F6P) fructose-1,6-bisphosphate (F1, 6BP), 3-phosphoglycerate (3-PG), 2-phosphoglycerate (2-PG), 2,3-bisphosphoglycerate (2,3-BPG), glyceraldehyde-3-phosphate (G3P), dihydroxyacetone phosphate (DHAP) and phosphoenolpyruvate (PEP). It should be noted that each target molecule has at least one phosphorus.

According to the present invention, the target molecules are selected from the group of metabolic substrates of the gluconeogenesis and glycolysis process.

More specifically, according to the present invention, the target molecules are selected from the group of glucose-6-phosphate (G6P), fructose-6-phosphate (F6P) fructose-1,6-bisphosphate (F1, 6BP), 3-phosphoglycerate (3-PG), 2-phosphoglycerate (2-PG), 2,3-bisphosphoglycerate (2,3-BPG), glyceraldehyde-3-phosphate (G3P), dihydroxyacetone phosphate (DHAP) and phosphoenolpyruvate (PEP).

According to the present invention, in the biological object 3, e.g. in a person or animal or an *in vitro* sample having cells of interest, each type of target molecule is present at a respective concentration such that the concentration of phosphorus ³¹P bound in the target molecules of a specific target type is less than 500 µM.

Preferably, each type of target molecule is present at a respective concentration such that the concentration of phosphorus ³¹P bound in the target molecules of a specific target type is even less than 100 µM.

It should be noted that, to date, there are no magnetic resonance imaging machines 1 operating with magnetic fields greater than 7T in the clinical setting. Therefore, to date, it is not possible to detect phosphorus ³¹P bound in target molecules and, therefore, it is not possible to detect the respective response signals with the MRI machines available in the clinical field, and, consequently, it is not possible to study metabolic processes, in particular enzymatic processes such as glycolysis without using exogenous contrast agents.

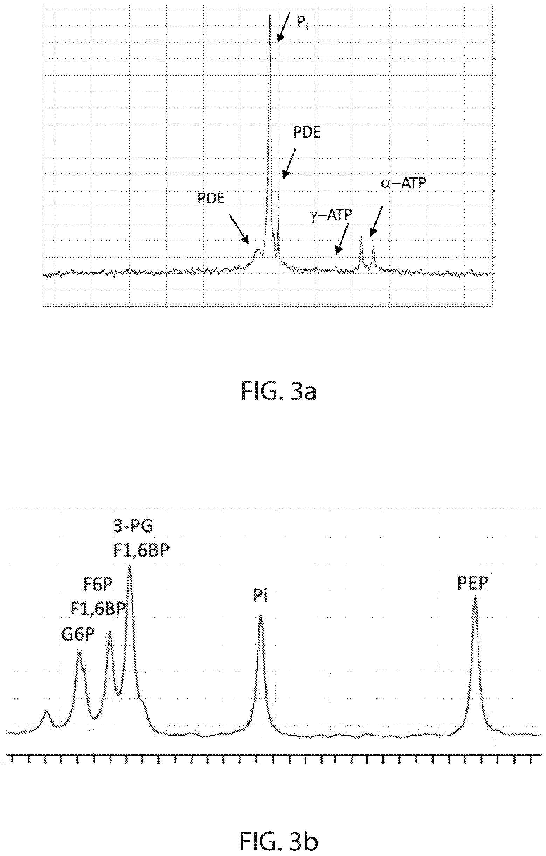

For example, with reference to Figure 3a, a phosphorus response spectrum ³¹P can be seen, obtained in an *in vitro* sample of a TS/A murine breast cancer cell line suspended in Hepes at pH 7 and 310K and obtained in a static magnetic field of 14T. Figure 3b shows a phosphorus ³¹P response spectrum obtained in an *in vitro* sample in a solution containing glucose-6-phosphate (G6P), fructose 1,6 bisphosphate (F1, 6BP), fructose-6-phosphate (F6P), 3-phosphoglyceric acid (3-PG), inorganic phosphate (Pi) and phosphoenolpyruvate (PEP) all at a concentration of 10 mM at pH 7 and 310K and in a static magnetic field of 14T. With the exception of the inorganic phosphate Pᵢ none of these signals can be seen in the ³¹P NMR spectrum of Figure 3a because their respective concentrations in the cells are too low to give rise to a detectable signal.

The present invention makes it possible, without the use of any contrast agent to be injected, to study and analyze metabolic processes, in particular enzymatic processes such as glycolysis also with a magnetic resonance imaging machine 1 operating with clinical and pre-clinical static magnetic fields.

It should be noted that the respective resonance of the phosphorus ³¹P bound in a first type of target molecule is different from the respective resonance if the phosphorus ³¹P is bound in another type of target molecule. Therefore, it is possible to selectively saturate the phosphorus ³¹P bound in different types of target molecules.

In addition, a rf field with a specific frequency must be used for each type of target molecule in order to saturate the phosphorus ³¹P bound in the target molecules of the specific type.

For example, in order to saturate the phosphorus ³¹P bound in glucose-6-phosphate (G6P), a different rf-field would have to be used with respect to the case of saturating the phosphorus ³¹P bound in glyceraldehyde-3-phosphate (G3P).

Therefore, by selectively choosing an RF field, it is possible to obtain the respective information about the specific target molecule and to analyze the respective reactions.

In the following, the inventive method is described in more detail.

The inventive method is a magnetic resonance imaging method for analyzing metabolic reactions, that belong to glycolysis and/or gluconeogenesis, in the biological object 3 (of interest) .

The biological object 3 can be a living object such as a person or an animal, but also an *in vitro* sample containing cells of interest.

The method comprises the steps of:
- placing the biological object 3 in a measurement space 2;
- generating a static magnetic field in the measurement space 2;
- defining one or more types of target molecules, each target molecule having at least one phosphorus ³¹P bound in the target molecule itself;
- saturating, during which a sequence of at least one or more selective radiofrequency pulses is emitted to saturate the phosphorus ³¹P bound in the target molecules;
- emitting one or more nonselective radiofrequency pulses to excite the nuclear spins of the atoms of the phosphorus ³¹P, particularly in a frequency range of interest;
- acquiring, following the step of emitting, nuclear magnetic resonance response signals belonging to endogenous phosphorylated molecules containing phosphorus in exchange with the saturated phosphorus ³¹P of the target molecules; and
- determining from the response signals, particularly from an intensity of the response signals, a respective saturation transfer value from the phosphorus ³¹P of the target molecules to the endogenous phosphorylated molecules.

In particular, this method operates in CEST mode.

In particular, the step of emitting is performed following the step of saturating.

According to the invention, the method also involves performing the step of emitting and the step of acquiring without performing the step of saturating, particularly in order to be able to compare the results obtained with and without saturation.

In other words, the inventive method involves two steps. In one step, the step of emitting and the step of acquiring are executed without the step of saturating being executed, and in the other step, the step of emitting and the step of acquiring are executed following the execution of the step of saturating. The comparison of the response signals allows the determination of a measure of the magnitude of the saturation transfer from the ratio between the response signals, specifically between the intensities of the signals, obtained with and without saturation, or obtained by saturating two points symmetrical to the response signals, one of which corresponds to the signal at the frequency of the target molecule.

In particular, the z spectrum is a graph in which the intensity of the response signal is plotted as a function of the selectively radiated frequency. From the z spectrum, the magnitude of the saturation transfer can be measured by calculating the ratio of the intensity of the response signal when the phosphorus ³¹P resonance frequency of the target molecule is saturated to the intensity of the response signal when a different frequency is saturated or to a response signal in the absence of saturation.

The method described here can also be used to obtain a reference Z spectrum that can then be used as a reference for subsequent measurements; in other words, the method can be used to define a fingerprint.

For example, a new z spectrum can be compared with the reference z spectrum. The new z spectrum is obtained by running the method after a defined time has elapsed after the determination of the reference z spectrum and considering the same biological object 3 and the same region under analysis.

Alternatively or additionally, the determination of the reference z spectrum can result from applying the method to a first biological object 3 and determining the other z spectrum from a measurement on a second biological object 3 (being of the same genus - e.g. first and second biological objects 3 are both animals or both humans, etc.).

More specifically, the biological object 3 can be a person, an animal or an *in vitro* sample containing cells of interest.

More specifically, the method can be applied to analyze one or more sections of the biological object 3.

Preferably, but not necessarily, the method can be applied to obtain three-dimensional information of the saturation transfer value of saturated phosphorus ³¹P nuclei.

In more detail, the static magnetic field is generated by the generating device 4 during the generation step.

In more detail, radiofrequency pulses are emitted by the emitter device 5 during the saturation step.

More specifically, radiofrequency pulses with a spectrum of frequencies are emitted during the saturation step in order to saturate the phosphorus ³¹P that is bound in the target molecules.

Preferably but not necessarily, radiofrequency pulses can be emitted that can saturate the nuclear spins of phosphorus ³¹P atoms bound in target molecules of different types. In this case, the spectrum of the RF pulses must be chosen in order to saturate the nuclear spins of the phosphorus atoms ³¹P bound in the target molecules of the different types.

It should be noted that during the saturation of phosphorus ³¹P the respective core is saturated.

In particular, selective RF pulses interact with their respective nuclear spins.

It should be noted that phosphorus ³¹P saturation is a physical change, but has no chemical consequence.

The process of saturation transfer is also known in the industry to be described with reference to signals. Specifically, the saturation of phosphorus ³¹P bound in the target molecules is described in the sense of a saturated signal and the saturation transfer is described in the sense that the saturated signal is transferred to the response signals of an endogenous phosphorylated molecule containing phosphorus in exchange with the saturated ³¹P.

It should be noted that response signals are acquired during the execution of the method.

In more detail, during the step of acquiring, the response signals from endogenous phosphorylated molecules being in saturation transfer with saturated ³¹P are acquired.

In particular, the step of emitting is performed to stimulate the response signals.

Specifically, during the step of emitting, one or more (non-selective) radiofrequency pulses of excitation are emitted to excite phosphorus ³¹P response signals from the endogenous phosphorylated molecule that has a phosphorus in saturation transfer with the saturated phosphorus ³¹P.

In particular, (non-selective) radiofrequency excitation pulses are required to excite all the phosphorus ³¹P nuclei in the range of interest.

Specifically, excitation radiofrequency pulses are emitted by the emitter device 5.

In more detail, during the step of acquiring, the excitation radiofrequency pulses are included in the frequency range of the endogenous phosphorylated molecules, specifically placing the signal to which the saturation will be transferred (i.e. the response signals) in the centre of the response spectrum.

The step of acquiring consists of detecting the signal from the biological object 3 after excitation of the phosphorus ³¹P nuclei.

The Applicant found that it is advantageous to choose the endogenous phosphorylated molecule leading to the response signals in the group of free inorganic phosphate (Pᵢ), adenosine triphosphate (ATP) and phosphocreatine (Pcr).

In particular, the free inorganic phosphate Pi and adenosine triphosphate are in exchange with the saturated phosphorus ³¹P in the target molecule.

In addition, phosphocreatine (PCr) is also in exchange with phosphorus ³¹P saturated in the target molecule.

It is to be stressed that during the step of acquiring, the response signals are chosen to be different from those coming from water.

In addition, the step of saturating, the step of emitting, the step of acquiring and the step of determining are performed for more than one target molecule.

According to a variant, a plurality of selective radiofrequency pulses are emitted during the saturation step at different offset frequencies with respect to the resonance frequency of the response signals.

Additionally or alternatively, a plurality of saturation radiofrequency pulses are emitted during the saturation step, including at least a first selective radiofrequency pulse and at least a second selective radiofrequency pulse. The first selective radiofrequency pulse has a first frequency that corresponds to a first resonance frequency of the phosphorus ³¹P bound to the target molecules and that differs by one value from a second resonance frequency that corresponds to the response signals. The second selective radiofrequency pulse has a third frequency that is distanced from the second resonance frequency by a value that corresponds to the difference value with the opposite sign.

Additionally or alternatively, a plurality of selective radiofrequency pulses are emitted during the saturation step, including at least one selective radiofrequency pulse with a first frequency that corresponds to a first resonance frequency of the phosphorus ³¹P bound in the target molecules and at least one other selective radiofrequency pulse with a second frequency that differs from the resonance frequency of the response signals. In particular, the second frequency is at least 10ppm away from the resonance frequency.

Preferably but not necessarily, the method also comprises a step of transforming during which the saturation transfer value is transformed into an indicator of a state of the metabolic process.

Preferably but not necessarily, the step of saturating, the step of emitting, the step of acquiring and the step of determining, and more specifically also the step of transforming, are performed for different zones of the biological object 3 in such a way as to obtain three-dimensional and localised information regarding the saturated phosphorus transfer value ³¹P saturated for each zone.

In particular, the step of saturating, the step of emitting, the step of acquiring and the step of determining are performed in the presence of magnetic field, in particular generated by the magnetic gradient device 8, that allow spatial localization of the response signals within the biological object 3 to obtain spatially encoded information relative to the phosphorus saturation transfer value of the saturated phosphorus ³¹P.

Preferably, but not necessarily, each saturation transfer value is converted into a color during the step of determining, in particular according to a defined color scale.

Advantageously, the method also includes a display step; each saturation transfer value is displayed, in particular by the display device 7, in terms of its respective colour, and in particular in three-dimensions.

Preferably, but not necessarily, a colorimetric (and three-dimensional) map is generated during the display step.

Advantageously, the method also includes a step of acquisition, during which an anatomical image of at least one section of the biological object 3 is acquired by proton magnetic resonance imaging.

Advantageously, during the display step, the anatomical image is displayed and the color map obtained by converting saturation transfer values into colors is superimposed on the anatomical image. In this way, it is possible to directly associate the various zones in the anatomical image with their respective transfer value.

Next, we demonstrate the effectiveness of the method described, using simple *in vitro* and *in vivo* examples.

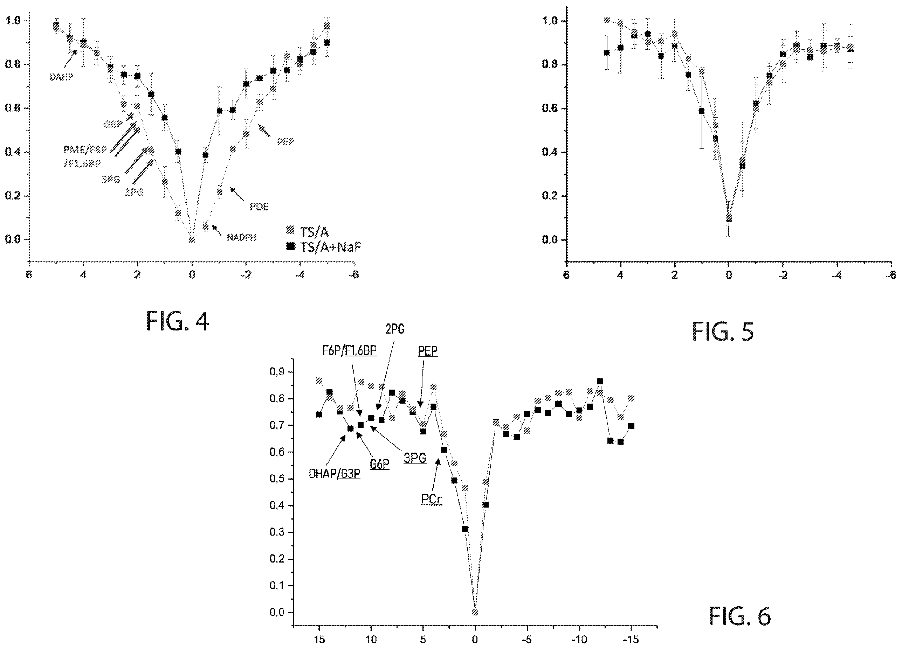

Figure 4 shows the results of response signal measurements represented in terms of its normalized intensity as a function of saturated frequency (known as the z spectrum). The two results shown in Figure 4 were obtained on a TS/A sample in Hepes/NaCl buffer at 310K and pH 7 with (black square) or without (gray square) the addition of sodium fluoride (NaF), which is a glycolysis inhibitor. The measurements were performed with a Bruker magnetic resonance imaging machine using a static magnetic field of 14 T, with an irradiation time of 2 seconds at an irradiation power of **3** µT. Each point was obtained from 64 scans per point.

There is a clear difference in the results depending on the activity of the glycolysis process, which highlights the applicability of the proposed method.

Figure 5 shows the results obtained on a sample of three healthy mice (gray square) and a sample consisting of three healthy mice that were given a dose of 0.36 mmol/Kg NaF (black square). The difference between the two spectra is statistically significant precisely in the chemical shift region related to the glycolysis substrates. The results were obtained using a Bruker Pharmascan magnetic resonance imaging machine with a static magnetic field of 7 T, an irradiation time of 5s at a power of 1µT and 64 scans per point.

Figure 6 shows the comparison between z spectra (intensity of the acquired signal versus the frequency of the saturated signal of the target molecule) acquired on the ATP signal referring to a portion of the image located on the liver of a healthy mouse (black square) and a mouse suffering from biliary tract disease that causes liver fibrosis in the early stages of onset (gray square). The difference between the two spectra is statistically significant in the chemical shift region related to the glycolysis substrates. The results were obtained using a Bruker Pharmascan magnetic resonance imaging machine with a static magnetic field of 7 T, an irradiation time of 2s at a power of 2µT and 1 scan per point, using a localized spectroscopy sequence. The voxel located in the liver has a volume of 1.6 ml.

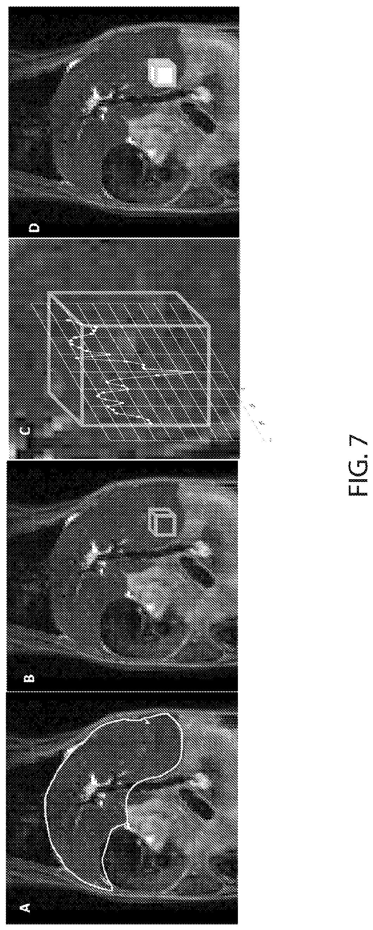

Figures 7A to 7D show results obtained from the analysis of a mouse liver. Figure 7A illustrates an anatomical image. The additional line delimits the region in the image that corresponds to the extension of the liver. Figure 7B schematically illustrates a voxel (i.e. a three-dimensional area) located at the liver. Figure 7C represents an enlargement of the voxel in Figure 7B and also illustrates the z spectrum obtained for this voxel. Figure 7D illustrates the coding of the saturation transfer value in terms of a color.

The magnetic resonance imaging machine 1 also comprises a computer program product, loadable into a memory of the control device 6 of the magnetic resonance imaging machine and including suitable instructions for performing the steps of the method according to the present invention and as described above.

From an examination of the characteristics of the method realized according to the present invention, the advantages that can be achieved are evident.

The method is also applicable when the magnetic resonance signal intensity of the phosphorus ³¹P bound in the target molecules is below the signal-to-noise ratio for its acquisition with the conventional nuclear magnetic resonance spectroscopy technique and at static magnetic fields not exceeding 7T.

Furthermore, the inventive method can be applied to situations in which the concentration of phosphorus ³¹P bound to a target molecule type is less than 500 µM.

Furthermore, compared to the prior art, the inventive method allows endogenous phosphorylated molecules to be displayed, reducing the health risks associated with the administration of exogenous contrast agents.

Moreover, the method allows processes to be observed dynamically with a higher detection threshold than the magnetic resonance spectroscopy technique.

Moreover, the method allows several target molecules to be observed and distinguished simultaneously.

Finally, it is clear that modifications and variations can be made to the method described and shown herein without thereby departing from the scope of protection defined by the claims.

## Claims

1. **-** Magnetic resonance imaging method for analyzing metabolic processes in a biological object (3) and that belong to glycolysis and/or gluconeogenesis;
the method comprises the steps of:
- placing the biological object (3) in a measurement space (2);
- generating a static magnetic field in the measurement space (2);
- defining one or more types of target molecules, each target molecule having at least one phosphorus³¹ P bound in the target molecule itself;
- saturating, during which a sequence of at least one or more selective radiofrequency pulses is emitted to saturate the phosphorus³¹ P bound in the target molecules;
- emitting, one or more nonselective radiofrequency pulses to excite the nuclear spins of the atoms of the phosphorus P³¹;
- acquiring, following the step of emitting, nuclear magnetic resonance response signals belonging to endogenous phosphorylated molecules containing phosphorus in exchange with the saturated phosphorus ³¹P of the target molecules; and
- determining from the response signals, particularly from an intensity of the response signals, a respective saturation transfer value from the phosphorus ³¹P of the target molecules to the endogenous phosphorylated molecules;
wherein the method involves executing the step of emitting and the step of acquiring without the step of saturating being executed, and the method also involves the execution of the step of emitting and the step of acquiring following the execution of the step of saturating;
in which the target molecules are selected from the group of metabolic substrates of the gluconeogenesis and glycolysis process;
wherein the method is **characterized in that**
the concentration of the phosphorus ³¹P bound in the target molecules is less than 500 µM; and
**in that** the target molecules are selected from the group of glucose-6-phosphate (G6P), fructose-6-phosphate (F6P) fructose-1,6-bisphosphate (F1, 6BP), 3-phosphoglycerate (3-PG), 2-phosphoglycerate (2-PG), 2,3-bisphosphoglycerate (2,3-BPG), glyceraldehyde-3-phosphate (G3P), dihydroxyacetone phosphate (DHAP) and phosphoenolpyruvate (PEP) .

2. **-** Method according to claim 1, also including a step of transforming, during which the transfer value is transformed into an indicator of a state of the metabolic process.

3. **-** Method according to any one of the preceding claims, in which during the step of acquiring, the type of endogenous phosphorylated molecule from which the response signals results is that of free inorganic phosphate or that of adenosine triphosphate (ATP) or that of phosphocreatine (PCr).

4. **-** Method according to any one of the preceding claims, in which during the saturation step the following are emitted:
- a plurality of selective radio frequency pulses at different offset frequencies from the resonant frequency of the response signals; or
- at least one selective radiofrequency pulse with a first frequency that corresponds to a first resonance frequency of the phosphor ³¹P bound to the target molecule and that differs by a difference value from a second frequency that corresponds to the resonance of the response signals, and at least one other selective radiofrequency pulse with a third frequency that is spaced from the second resonance frequency by a value that corresponds to the difference value with opposite sign; or
- at least a first selective radiofrequency pulse with a first frequency that corresponds to a first resonance frequency of the phosphor ³¹P bound to the target molecule and that is different from the resonance frequency of the response signals, and at least a second selective radiofrequency pulse of the same intensity and duration as the first pulse having a second frequency that is spaced from the resonance frequency of the response signals.

5. **-** Method according to any one of the preceding claims, in which the step of saturating, the step of emitting, the step of acquiring and the step of determining are performed in the presence of magnetic field gradients that allow spatial localization of the response signals within the biological object (3) to obtain spatially encoded information relative to the phosphorus saturation transfer value of the saturated phosphorus ³¹P.

6. **-** Method according to any one of the preceding claims, in which during the step of determining each saturation transfer value is converted into a color step of determining;
in which the method also includes a step of displaying, during which each saturation transfer value is displayed in terms of its respective color;
in particular, a colorimetric map is generated during the step of displaying.

7. **-** Method according to claim 6, further comprising an step of acquisition, during which an anatomical image of a section of the biological object (3) is acquired by proton magnetic resonance imaging;
in which during the step of displaying the anatomical image is displayed and a colorimetric map obtained by converting the saturation transfer values into colors is superimposed on the anatomical image.

8. **-** Method according to any one of the preceding claims, in which **the** metabolic process is an enzymatic reaction.

9. **-** Method according to any one of the preceding claims, in which the step of saturating, the step of emitting, the step of acquiring and the step of determining are performed for more than one target molecule.

10. **-** Method according to any one of the preceding claims, in which, during the step of acquiring, radiofrequency pulses are emitted to excite phosphorus response signals ³¹P within the frequency range of endogenous phosphorylated molecules.

11. Method according to **any** one of the preceding claims, in which the intensity of the reported response signals as a function of radiated frequency defines a Z spectrum;
in which the method also involves comparing the obtained Z spectrum with a reference Z spectrum.

12. A computer program product loadable into a memory of a control device of a magnetic resonance imaging machine comprising instructions which when executed by the control device cause the magnetic resonance imaging machine to perform the steps of the method according to any one of claims 1 to 11.

13. Magnetic resonance imaging machine (1) for analyzing at least one section of a biological object (3) comprising:
- a measurement space (2);
- a generating device (4) configured to generate a static magnetic field in the measurement space (2);
- an emitter device (5) configured to emit radio frequency pulses;
- an acquisition device configured to acquire response signals (5); and
- a control device (6) comprising a computer program product according to claim 12.

## Patentansprüche

1. Magnetresonanzbildgebungsverfahren zur Analyse von Stoffwechselprozessen in einem biologischen Objekt (3), die zur Glykolyse und/oder Glukoneogenese gehören,
wobei das Verfahren folgende Schritte umfasst:
- Platzieren des biologischen Objekts (3) in einem Messraum (2);
- Erzeugen eines statischen Magnetfelds in dem Messraum (2);
- Definieren einer oder mehrerer Arten von Zielmolekülen, wobei jedes Zielmolekül mindestens ein Phosphor ³¹P aufweist, das in dem Zielmolekül gebunden ist;
- Sättigen, während dessen eine Abfolge von mindestens einem oder mehreren selektiven Hochfrequenzimpulsen ausgesendet wird, um das in den Zielmolekülen gebundene Phosphor ³¹P zu sättigen;
- Aussenden eines oder mehrerer nicht selektiver Hochfrequenzimpulse, um die Kernspins der Atome des Phosphors P³¹ anzuregen;
- Erfassen, im Anschluss an den Schritt des Aussendens, von Antwortsignalen der kernmagnetischen Resonanz, die zu endogenen phosphorylierten Molekülen gehören, die Phosphor im Austausch mit dem gesättigten Phosphor ³¹P der Zielmoleküle enthalten, und
- Bestimmen eines jeweiligen Sättigungsübertragungswerts vom Phosphor ³¹P der Zielmoleküle zu den endogenen phosphorylierten Molekülen anhand der Antwortsignale, insbesondere anhand einer Intensität der Antwortsignale, wobei das Verfahren das Ausführen des Schritts des Aussendens und des Schritts des Erfassens ohne Ausführung des Schritts des Sättigens umfasst und das Verfahren zudem die Ausführung des Schritts des Aussendens und des Schritts des Erfassens im Anschluss an die Ausführung des Schritts des Sättigens beinhaltet,
wobei die Zielmoleküle aus der Gruppe der Stoffwechselsubstrate des Glukoneogenese- und Glykolyseprozesses ausgewählt sind,
wobei das Verfahren **dadurch gekennzeichnet ist, dass**
die Konzentration des in den Zielmolekülen gebundenen Phosphors ³¹P weniger als 500 µM beträgt, und
dadurch dass die Zielmoleküle aus der Gruppe von Glucose-6-phosphat (G6P), Fructose-6-phosphat (F6P), Fructose-1,6-bisphosphat (F1, 6BP), 3-Phosphoglycerat (3-PG), 2-Phosphoglycerat (2-PG), 2,3-Bisphosphoglycerat (2,3-BPG), Glyceraldehyd-3-phosphat (G3P), Dihydroxyacetonphosphat (DHAP) und Phosphoenolpyruvat (PEP) ausgewählt sind.

2. Verfahren nach Anspruch 1, das zudem einen Schritt des Umwandelns einschließt, während dessen der Übertragungswert in einen Indikator für einen Zustand des Stoffwechselprozesses umgewandelt wird.

3. Verfahren nach einem der vorhergehenden Ansprüche, bei dem während des Schritts des Erfassens die Art des endogenen phosphorylierten Moleküls, von dem die Antwortsignale stammen, die von freiem anorganischem Phosphat oder die von Adenosintriphosphat (ATP) oder die von Phosphokreatin (PCr) ist.

4. Verfahren nach einem der vorhergehenden Ansprüche, bei dem während des Schritts des Sättigens Folgendes ausgesendet wird:
- eine Vielzahl von selektiven Hochfrequenzimpulsen bei unterschiedlichen Offset-Frequenzen gegenüber der Resonanzfrequenz der Antwortsignale oder
- mindestens ein selektiver Hochfrequenzimpuls mit einer ersten Frequenz, die einer ersten Resonanzfrequenz des an das Zielmolekül gebundenen Phosphors ³¹P entspricht und sich um einen Differenzwert von einer zweiten Frequenz unterscheidet, die der Resonanz der Antwortsignale entspricht, sowie mindestens ein weiterer selektiver Hochfrequenzimpuls mit einer dritten Frequenz, die von der zweiten Resonanzfrequenz um einen Wert beabstandet ist, der dem Differenzwert mit entgegengesetztem Vorzeichen entspricht, oder
- mindestens ein erster selektiver Hochfrequenzimpuls mit einer ersten Frequenz, die einer ersten Resonanzfrequenz des an das Zielmolekül gebundenen Phosphors ³¹P entspricht und sich von der Resonanzfrequenz der Antwortsignale unterscheidet, sowie mindestens ein zweiter selektiver Hochfrequenzimpuls mit derselben Intensität und Dauer wie der erste Impuls, der eine zweite Frequenz aufweist, die von der Resonanzfrequenz der Antwortsignale beabstandet ist.

5. Verfahren nach einem der vorhergehenden Ansprüche, bei dem der Schritt des Sättigens, der Schritt des Aussendens, der Schritt des Erfassens und der Schritt des Bestimmens in Gegenwart von Magnetfeldgradienten durchgeführt werden, die eine räumliche Lokalisierung der Antwortsignale innerhalb des biologischen Objekts (3) ermöglichen, um räumlich kodierte Informationen bezüglich des Phosphor-Sättigungsübertragungswerts des gesättigten Phosphors ³¹P zu erhalten.

6. Verfahren nach einem der vorhergehenden Ansprüche, bei dem während des Schritts des Bestimmens jeder Sättigungsübertragungswert in einen Farbschritt des Bestimmens umgewandelt wird,
wobei das Verfahren zudem einen Schritt des Anzeigens umfasst, während dessen jeder Sättigungsübertragungswert in Form seiner jeweiligen Farbe angezeigt wird,
wobei während des Schritts des Anzeigens insbesondere eine kolorimetrische Karte erzeugt wird.

7. Verfahren nach Anspruch 6, ferner umfassend einen Schritt des Erfassens, während dessen ein anatomisches Bild eines Teilabschnitts des biologischen Objekts (3) durch Protonen-Magnetresonanzbildgebung erfasst wird,
wobei während des Schritts des Anzeigens das anatomische Bild angezeigt wird und eine durch Umwandlung der Sättigungsübertragungswerte in Farben erhaltene kolorimetrische Karte auf das anatomische Bild überlagert wird.

8. Verfahren nach einem der vorhergehenden Ansprüche, bei dem der Stoffwechselprozess eine enzymatische Reaktion ist.

9. Verfahren nach einem der vorhergehenden Ansprüche, bei dem der Schritt des Sättigens, der Schritt des Aussendens, der Schritt des Erfassens und der Schritt des Bestimmens für mehr als ein Zielmolekül durchgeführt werden.

10. Verfahren nach einem der vorhergehenden Ansprüche, bei dem während des Schritts des Erfassens Hochfrequenzimpulse ausgesendet werden, um Phosphor-Antwortsignale ³¹P innerhalb des Frequenzbereichs endogener phosphorylierter Moleküle anzuregen.

11. Verfahren nach einem der vorhergehenden Ansprüche, bei dem die Intensität der gemessenen Antwortsignale als Funktion abgestrahlter Frequenz ein Z-Spektrum definiert,
wobei das Verfahren zudem das Vergleichen des erhaltenen Z-Spektrums mit einem Referenz-Z-Spektrum beinhaltet.

12. Computerprogrammprodukt, das in einen Speicher einer Steuervorrichtung einer Magnetresonanzbildgebungsmaschine geladen werden kann und Anweisungen umfasst, die, wenn sie durch die Steuervorrichtung ausgeführt werden, bewirken, dass die Magnetresonanzbildgebungsmaschine die Schritte des Verfahrens nach einem der Ansprüche 1 bis 11 durchführt.

13. Magnetresonanzbildgebungsmaschine (1) zur Analyse mindestens eines Teilabschnitts eines biologischen Objekts (3), umfassend:
- einen Messraum (2);
- eine Erzeugungsvorrichtung (4), die ausgelegt ist, um ein statisches Magnetfeld in dem Messraum (2) zu erzeugen;
- eine Aussendevorrichtung (5), die ausgelegt ist, um Hochfrequenzimpulse auszusenden;
- eine Erfassungsvorrichtung, die ausgelegt ist, um Antwortsignale (5) zu erfassen, und
- eine Steuervorrichtung (6), die ein Computerprogrammprodukt nach Anspruch 12 umfasst.

## Revendications

1. Procédé d'imagerie par résonance magnétique destiné à analyser les processus métaboliques d'un objet biologique (3) relevant de la glycolyse et/ou de la gluconéogenèse ;
le procédé comprend les étapes suivantes :
- placer l'objet biologique (3) dans un espace de mesure (2) ;
- générer un champ magnétique statique dans l'espace de mesure (2) ;
- - définir un ou plusieurs types de molécules cibles, chaque molécule cible comportant au moins un atome de phosphore ³¹P lié au sein de la molécule cible elle-même ;
- une étape de saturation, au cours de laquelle une séquence d'au moins une ou plusieurs impulsions radiofréquences sélectives est émise pour saturer le phosphore ³¹P lié dans les molécules cibles ;
- émettre une ou plusieurs impulsions radiofréquences non sélectives pour exciter les spins nucléaires des atomes du phosphore P³¹;
- acquérir, à la suite de l'étape d'émission, des signaux de réponse par résonance magnétique nucléaire appartenant à des molécules phosphorylées endogènes contenant du phosphore en échange avec le phosphore ³¹P saturé des molécules cibles ; et
- déterminer, à partir des signaux de réponse, en particulier à partir de l'intensité des signaux de réponse, une valeur de transfert de saturation respective du phosphore ³¹P des molécules cibles vers les molécules phosphorylées endogènes ; dans lequel le procédé implique l'exécution de l'étape d'émission et de l'étape d'acquisition sans que l'étape de saturation soit exécutée, et le procédé implique également l'exécution de l'étape d'émission et de l'étape d'acquisition à la suite de l'exécution de l'étape de saturation ;
dans lequel les molécules cibles sont choisies parmi le groupe des substrats métaboliques des processus de gluconéogenèse et de glycolyse ;
dans lequel le procédé est **caractérisé en ce que**
la concentration en phosphore ³¹P lié aux molécules cibles est inférieure à 500 µM ; et
**en ce que** les molécules cibles sont choisies parmi le groupe comprenant le glucose-6-phosphate (G6P), le fructose-6-phosphate (F6P), le fructose-1,6-bisphosphate (F1, 6BP), du 3-phosphoglycérate (3-PG), du 2-phosphoglycérate (2-PG), du 2,3-bisphosphoglycérate (2,3-BPG), du glycéraldéhyde-3-phosphate (G3P), du phosphate de dihydroxyacétone (DHAP) et du phosphoénolpyruvate (PEP).

2. Procédé selon la revendication 1, comprenant en outre une étape de transformation, au cours de laquelle la valeur de transfert est transformée en un indicateur d'un état du processus métabolique.

3. Procédé selon l'une quelconque des revendications précédentes, dans lequel, au cours de l'étape d'acquisition, le type de molécule phosphorylée endogène à l'origine des signaux de réponse est celui du phosphate inorganique libre, celui de l'adénosine triphosphate (ATP) ou celui de la phosphocréatine (PCr).

4. Procédé selon l'une quelconque des revendications précédentes, dans lequel, au cours de l'étape de saturation, sont émises :
- une pluralité d'impulsions de radiofréquence sélectives à différentes fréquences décalées par rapport à la fréquence de résonance des signaux de réponse ; ou
- au moins une impulsion de radiofréquence sélective ayant une première fréquence qui correspond à une première fréquence de résonance du phosphore ³¹P lié à la molécule cible et qui diffère, d'une valeur de différence, d'une deuxième fréquence correspondant à la résonance des signaux de réponse, et au moins une autre impulsion de radiofréquence sélective ayant une troisième fréquence qui est espacée de la deuxième fréquence de résonance d'une valeur correspondant à la valeur de différence avec un signe opposé ; ou
- au moins une première impulsion de radiofréquence sélective ayant une première fréquence qui correspond à une première fréquence de résonance du phosphore ³¹P lié à la molécule cible et qui est différente de la fréquence de résonance des signaux de réponse, et au moins une deuxième impulsion de radiofréquence sélective de même intensité et de même durée que la première impulsion, ayant une deuxième fréquence qui est espacée de la fréquence de résonance des signaux de réponse.

5. Procédé selon l'une quelconque des revendications précédentes, dans lequel l'étape de saturation, l'étape d'émission, l'étape d'acquisition et l'étape de détermination sont réalisées en présence de gradients de champ magnétique permettant la localisation spatiale des signaux de réponse au sein de l'objet biologique (3) afin d'obtenir des informations codées spatialement relatives à la valeur de transfert de saturation du phosphore ³¹P saturé.

6. Procédé selon l'une quelconque des revendications précédentes, dans lequel, au cours de l'étape de détermination, chaque valeur de transfert de saturation est convertie en une étape de couleur de détermination ;
dans lequel le procédé comprend également une étape d'affichage, au cours de laquelle chaque valeur de transfert de saturation est affichée en fonction de sa couleur respective ;
en particulier, une carte colorimétrique est générée au cours de l'étape d'affichage.

7. Procédé selon la revendication 6, comprenant en outre une étape d'acquisition, au cours de laquelle une image anatomique d'une section de l'objet biologique (3) est acquise par imagerie par résonance magnétique protonique ;
dans lequel, au cours de l'étape d'affichage, l'image anatomique est affichée et une carte colorimétrique obtenue en convertissant les valeurs de transfert de saturation en couleurs est superposée à l'image anatomique.

8. Procédé selon l'une quelconque des revendications précédentes, dans lequel le processus métabolique est une réaction enzymatique.

9. Procédé selon l'une quelconque des revendications précédentes, dans lequel l'étape de saturation, l'étape d'émission, l'étape d'acquisition et l'étape de détermination sont réalisées pour plus d'une molécule cible.

10. Procédé selon l'une quelconque des revendications précédentes, dans lequel, au cours de l'étape d'acquisition, des impulsions radiofréquences sont émises pour exciter des signaux de réponse du phosphore ³¹P dans la gamme de fréquences des molécules phosphorylées endogènes.

11. Procédé selon l'une quelconque des revendications précédentes, dans lequel l'intensité des signaux de réponse enregistrés en fonction de la fréquence rayonnée définit un spectre Z ;
dans lequel le procédé implique également la comparaison du spectre Z obtenu avec un spectre Z de référence.

12. Produit logiciel pouvant être chargé dans une mémoire d'un dispositif de commande d'un appareil d'imagerie par résonance magnétique, comprenant des instructions qui, lorsqu'elles sont exécutées par le dispositif de commande, amènent l'appareil d'imagerie par résonance magnétique à effectuer les étapes du procédé selon l'une quelconque des revendications 1 à 11.

13. Appareil d'imagerie par résonance magnétique (1) destiné à analyser au moins une section d'un objet biologique (3), comprenant :
- un espace de mesure (2) ;
- un dispositif de génération (4) configuré pour générer un champ magnétique statique dans l'espace de mesure (2) ;
- un dispositif émetteur (5) configuré pour émettre des impulsions de radiofréquence ;
- un dispositif d'acquisition configuré pour acquérir des signaux de réponse (5) ; et
- un dispositif de commande (6) comprenant un produit logiciel selon la revendication 12.
